# EUROPÄISCHE PATENTSCHRIFT

(11) **EP 1 100 793 B1**
(45) Veröffentlichungstag und Bekanntmachung des Hinweises auf die Patenterteilung: **18.09.2002**
(21) Anmeldenummer: 99932864.4
(22) Anmeldetag: 12.07.1999
(51) Int. Cl.: C07D 339/04, C07B 57/00, A61K 31/385, A23L 1/30

(54) **KRISTALLMODIFIKATION DER LIPONSÄURE**
CRYSTAL MODIFICATION OF LIPOIC ACID
MODIFICATION DE L'ACIDE LIPOIQUE

(30) Priorität: 31.07.1998 DE 19834608
(43) Veröffentlichungstag der Anmeldung: 23.05.2001
(73) Patentinhaber: BASF AKTIENGESELLSCHAFT, 67056 Ludwigshafen (DE)
(72) Erfinder: KLATT, Martin, Jochen, D-67098 Bad Dürkheim (DE); NIEBEL, Markus, D-68163 Mannheim (DE); PAUST, Joachim, D-67141 Neuhofen (DE); RIEGER, Jens, D-67069 Ludwigshafen (DE)
(86) Internationale Anmeldenummer: EP9904870
(87) Internationale Veröffentlichungsnummer: WO00008012

(56) Entgegenhaltungen:
- EP-A- 0 543 088
- EP-A- 0 593 896

## Beschreibung

Die Erfindung betrifft Verfahren zur Herstellung von kristalliner α-Liponsäure mit hohen Ausbeuten, eine neue Kristallmodifikation von α-Liponsäure und deren Verwendung.

α-Liponsäure wird in der Lebensmittelindustrie und in pharmazeutischen Formulierungen eingesetzt.

Wenn im folgenden von Liponsäure die Rede ist, sind immer die enantiomerenreinen Verbindungen ((R)- oder (S)-Liponsäure) zu verstehen.

Verfahren zur Herstellung von enantiomerenreiner Liponsäure sind z.B. in Bulman-Page et al., J. Chem. Soc., Chem. Commun. 1986, 1409, beschrieben. Des weiteren kann das Racemat sehr effektiv nach dem in EP 0 586 987 beschriebenen Verfahren hergestellt werden. Die Enantiomerentrennung kann durch Verwendung von chiralen Aminen erfolgen (z.B. EP 0 543 088). (R)-Liponsäure kann auch aus natürlichen Quellen isoliert werden (Reed et al. JACS 1953 Vol. 75, 1267). (R)-Liponsäure kann außerdem durch das in EP 487 986 genannte Verfahren enantiomerenrein hergestellt werden.

Das Rohprodukt wird anschließend in Pentan, Hexan oder Cyclohexan umkristallisiert.

Alternativ kann das Rohprodukt auch in Cyclohexan/Essigester 2:1 umkristallisiert werden (EP 0 593 896).

Falls die genauen Umkristallisationsbedingungen und Ausbeuten angegeben werden, wird aus Cyclohexan bei 5 bis 10°C 40 %, bezogen auf das Rohprodukt, und im Cyclohexanessigestergemisch 31 % bei ca. -15°C, bezogen auf das Rohprodukt, erhalten.

Die bisherigen Verfahren zur Herstellung von reiner kristalliner (R)- oder (S)-α-Liponsäure ergeben Ausbeuten, die für eine technische Herstellung von α-Liponsäure ungünstig sind.

Die Möglichkeit, die Ausbeute durch Weiterverwendung der Mutterlauge für die Kristallisation zu erhöhen, ist technisch aufwendig und führt im allgemeinen zu einer Erhöhung der Verunreinigungen, die bei pharmazeutischer Verarbeitung oder in der Lebensmittelindustrie hoch unerwünscht sind.

Aufgabe der vorliegenden Erfindung war die Entwicklung eines Verfahrens zur Herstellung von reiner, pharmazeutisch einsetzbarer Liponsäure in einer für die pharmazeutische Verarbeitbarkeit günstigen Form.

Die bisher erzeugten Kristallisate weisen die von Reed et al. für (+)-alpha-Liponsäure aus Cyclohexan und die in EP 0 593 896 (aus Cyclohexan/Essigester) publizierten Röntgen-Diffraktogramme auf [Transmissions-Röntgen Diffraktometer Aufnahmen mit Cu K_{α1"} Strahlung (2 Theta)].

Die erfindungsgemäße Aufgabe wurde nun so gelöst, daß α-Liponsäure in einem organischen Lösungsmittel mit einer Dielektrizitätskonstante epsilon von 1,95 bis 2,4 gelöst wird. Die organische α-Liponsäurelösung wird dann auf 0 bis -20°C abgekühlt, wobei für enantiomerenreine α-Liponsäure ein neuartiges Kristallisat entsteht, das ein Röntgen-Diffraktogramm mit einer neuartigen Linienintensitätsverteilung sowie für die pharmazeutische Verarbeitung geeignet ist. Darüber hinaus wird die erfindungsgemäße Ware bei hoher Reinheit (mit Verunreinigung <0,1 %) in Ausbeuten von 75 % und mehr gewonnen. Die erfindungsgemäße Weise eignet sich sowohl für die pharmazeutische Verwendung als auch in der Lebensmittelindustrie, bei der Liponsäure als Zusatz zu Lebensmitteln verwendet wird, wie auch in Nahrungsergänzungspräparaten (Dietary Supplements).

Das erfindungsgemäße Kristallisat weist als intensivste Linie im 2 Θ-Diffraktogramm zwischen 15 und 30° eine Linie bei 23° aus. Die erfindungsgemäßen Kristallisate weisen ein Intensitätsverhältnis zwischen 2 Θ = 23° zu 2 Θ = 18,2° von mindestens 1 auf.

Die Verwendung der erfindungsgemäßen Lösungsmittel mit einer Dielektrizitätskonstante von 1,95 bis 2,4 haben darüber hinaus gegenüber den literaturbeschriebenen den Vorteil, daß sie bei der Kristallisation ein sehr reines Kristallisat bei hohen Ausbeuten liefern. Als Lösungsmittel oder Komponenten von Lösungsmittelgemischen können beispielsweise dienen: geradkettige oder verzweigte, gesättigte oder ein- oder mehrfach ungesättigte aliphatische Kohlenwasserstoffe mit einer Kettenlänge von C₅ bis C₈, cycloaliphatische Kohlenwasserstoffe wie Cyclopentan, Cyclohexan, Methylcyclohexan, ein- oder mehrfach halogenierte, bevorzugt chlorierte Kohlenwasserstoffe, mit einer Kettenlänge von 1 bis 4 Kohlenstoffatomen. Es können sowohl Lösungsmittelgemische wie technisches Hexan oder Heptan eingesetzt werden, bei denen Hexan oder Heptan die Hauptkomponente ist. Ebenso können auch die reinen Lösungsmittel eingesetzt werden.

Bevorzugt sind Lösungsmittelgemische aus aliphatischen und aromatischen Kohlenwasserstoffen.

Des weiteren kommen als Lösungsmittel oder Komponenten von Lösungsmitteln ein- und mehrkernige Aromaten, die gegebenenfalls substituiert sein können, in Frage. Genannt seien beispielsweise Toluol, o-, m- und p-Xylol, Ethyl-, Propyl- und Isopropylbenzol und Mesitylen.

Die erfindungsgemäßen Lösungsmittel oder Lösungsmittelgemische müssen außerdem einen Festpunkt unter 0°C, bevorzugt unter -20°C, besitzen, besonders bevorzugt unter -40°C. Bevorzugt sind außerdem Lösungsmittel, die möglichst wenig toxikologisch bedenklich sind, da die resultierende Liponsäure als Arzneimittel oder Lebensmittelzusatzstoff verwendet werden soll.

Die bevorzugte Obergrenze für die Dielektrizitätskonstante ist 2,2, besonders bevorzugt ist ein Bereich zwischen 2,0 und 2,1.

Die bevorzugte Obergrenze für den Temperaturbereich der Auskristallisation liegt bei -5°C, besonders bevorzugt bei -10°C.

Das Verfahren kann auch bei niedrigen Temperaturen durchgeführt werden, solange das Lösungsmittel oder Lösungsmittelgemisch nicht fest wird. Eine bevorzugte Temperaturuntergrenze liegt bei -20°C.

Bevorzugte Lösungsmittelgemische sind solche aus Toluol oder Xylol mit C₅-C₇-Aliphaten im Volumenverhältnis 1:1 bis 1:4, besonders bevorzugt ist ein Gemisch aus Toluol und technischem Hexan oder Toluol und technischem Heptan.

Die Liponsäure wird aus einem Lösungsmittel oder Lösungsmittelgemisch umkristallisiert, wobei das Gewichtsverhältnis zwischen Liponsäure und Lösungsmittel bevorzugt zwischen 1:5 und 1:15, besonders bevorzugt zwischen 1:8 und 1:12 liegt.

Bevorzugt wird bei einem Lösungsmittelgemisch erst in einer polaren Mischung oder in der polaren Lösungsmittelkomponente gelöst und dann die restliche unpolare Lösungsmittelkomponente für die Umkristallisation vor dem Abkühlen auf 0 bis -20°C beigefügt.

Die erfindungsgemäße Ware besitzt in wäßriger Lösung (1 g Liponsäure in 20 ml 1N NaOH) eine Extinktion von <0.300 (430 nm), Schichtdicke 1 cm.

### Beispiel 1

10 g (R)-α-Liponsäure wurden in 67 ml Lösungsmittelgemisch Toluol/techn. Hexan 1:1 bei 50°C eingetragen. Man erhält eine Lösung, die abfiltriert wird. Das Filtrat wurde mit 33 ml Hexan (techn.) gewaschen. Die vereinigten Lösungen wurden 1 h im Eiswasser bei 0 bis 5°C gehalten und anschließend 1 h bei ca. -15°C mit Eis/Kochsalzgemisch-Kühlung nachgerührt.
Anschließend wurde abgesaugt und im Vakuum bei Raumtemperatur getrocknet: 8,1 g (R)-(+)-α-Liponsäure

### Beispiel 2

10 g rohe (R)-α-Liponsäure werden in 35 ml Toluol von 50°C gelöst. Man setzt 35 ml techn. Heptan zu, filtriert die Lösung bei 50°C über 4 g Kieselgel F 60 und setzt dem Filtrat weitere.35 ml techn. Heptan zu. Die Lösung wird auf 0 bis 5°C gekühlt, geimpft und nach 1 h mit einer Abkühlrate von 5°C/h auf -15°C gekühlt.

Man isoliert das Wertprodukt durch Absaugen auf einer Fritte; wäscht zweimal mit 15 ml techn. Heptan und trocknet das Kristallisat im Stickstoffstrom: 8,0 g (R)-α-Liponsäure, Fp. 47,9 bis 48,9°C; [α]²⁴_{D} = 113,7°C, c = 1 in Benzol, Gehalt nach GC-Analyse 99,95 %; alle Spurenkomponenten <0,05 F1.-%.

Die erfindungsgemäß hergestellten Liponsäuren wurden einer Röntgenbeugungsanalyse mit Cu K-alpha-Strahlung unterzogen.

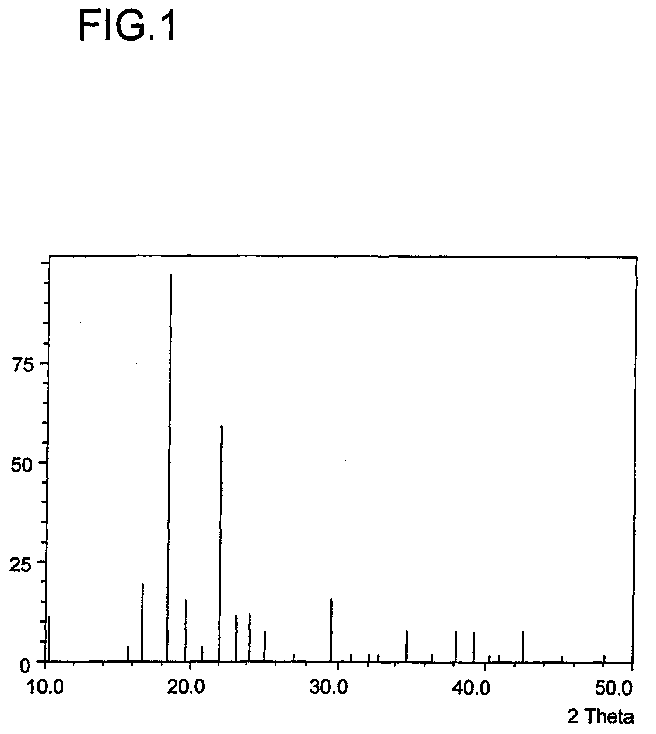

Figur 1 zeigt ein Pulverdiffraktogramm einer (R)-Liponsäure gemäß dem Stand der Technik aus Cyclohexan.

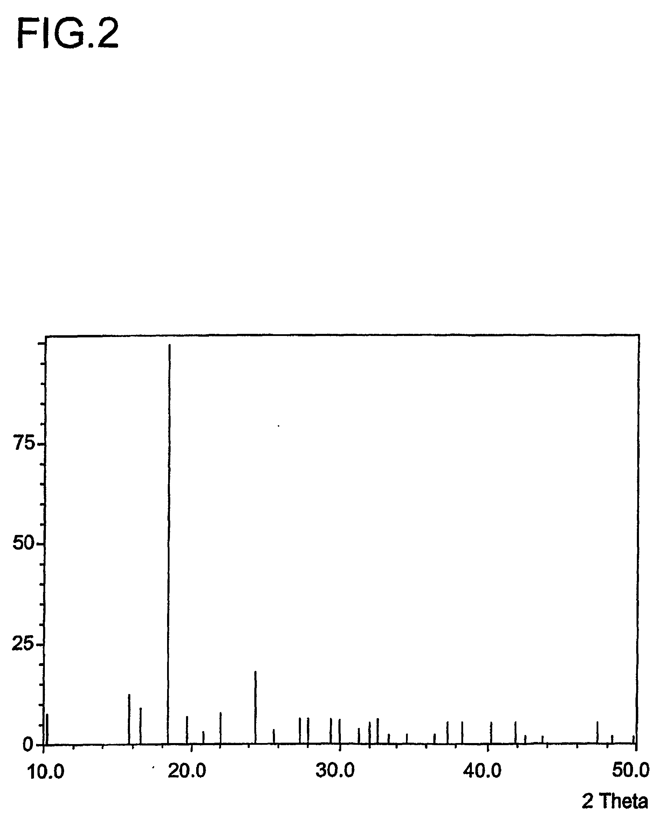

Figur 2 zeigt ein Pulverdiffraktogramm einer (R)-Liponsäure gemäß dem Stand der Technik aus Cyclohexan/Essigester.

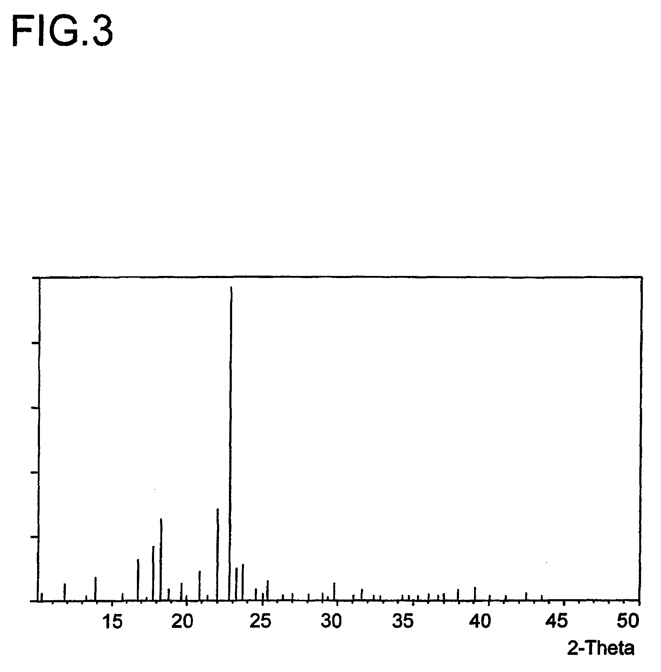

Figur 3 zeigt ein Pulverdiffraktogramm der erfindungsgemäßen (R)-Liponsäure.

## Patentansprüche

1. Enantiomerenreine kristalline R- oder S-Liponsäure, wobei die Reflexionslinien bei 2 Θ = 23° die intensivste im Bereich zwischen 15° und 30° im 2 Θ-Diffraktogramm ist.

2. Verfahren zur Herstellung kristalliner Liponsäure, wobei man die Liponsäure aus einem Lösungsmittel oder Lösungsmittelgemisch bei 0°C bis -20°C auskristallisiert und das Lösungsmittel oder das Lösungsmittelgemisch eine Dielektrizitätskonstante epsilon zwischen 1,95 und 2,4 aufweist.

3. Verfahren nach Anspruch 2, wobei ein Lösungsmittelgemisch aus aliphatischen und aromatischen Kohlenwasserstoffen verwendet werden.

4. Verfahren nach Anspruch 3, **dadurch gekennzeichnet, daß** als Lösungsmittelgemisch ein Gemisch aus Toluol und Hexan oder ein Gemisch aus Toluol und Heptan eingesetzt wird.

5. R-Liponsäure, erhältlich nach einem der Ansprüche 3 oder 4.

6. Liponsäure, erhältlich nach einem der Ansprüche 2 bis 4.

7. Verwendung von Liponsäure nach Anspruch 1, 5 oder 6 zur Herstellung von Arzneimitteln.

8. Verwendung von Liponsäure nach Anspruch 1, 5 oder 6 als Zusatz in Lebensmitteln oder als Komponente in Nahrungsergänzungspräparaten.

## Claims

1. Enantiomerically pure crystalline R- or S-lipoic acid for which the reflection line at 2 Θ = 23° is the most intense in the range from 15° to 30° in the 2 Θ diffractogram.

2. A process for preparing crystalline lipoic acid, which comprises crystallizing the lipoic acid at from 0°C to -20°C from a solvent or solvent mixture which has a dielectric constant ε of from 1.95 to 2.4.

3. A process as claimed in claim 2, wherein a solvent mixture of aliphatic and aromatic hydrocarbons is used.

4. A process as claimed in claim 3, wherein the solvent mixture used is a mixture of toluene and hexane or a mixture of toluene and heptane.

5. R-lipoic acid obtainable as claimed in claim 3 or 4.

6. Lipoic acid obtainable as claimed in any of claims 2 to 4.

7. The use of lipoic acid as claimed in claim 1, 5 or 6 for preparing drugs.

8. The use of lipoic acid as claimed in claim 1, 5 or 6 as a food additive or as a component of dietary supplements.

## Revendications

1. Acide R- ou S-lipoïque cristallisé, pur du point de vue des énantiomères, dans lequel la ligne de réflexion à 2 Θ = 23° est la plus intense dans l'intervalle entre 15° et 30° dans le diffractogramme 2 Θ.

2. Procédé pour la préparation d'acide lipoïque cristallisé, dans lequel on cristallise l'acide lipoïque dans un solvant ou un mélange de solvants entre 0°C et -20°C et le solvant ou le mélange de solvants présente une constante diélectrique epsilon entre 1,95 et 2,4.

3. Procédé selon la revendication 2, dans lequel on utilise un mélange de solvants composé d'hydrocarbures aliphatiques et aromatiques.

4. Procédé selon la revendication 3, dans lequel on utilise comme mélange de solvants un mélange de toluène et d'hexane ou un mélange de toluène et d'heptane.

5. Acide R-lipoïque, pouvant être obtenu selon l'une des revendications 3 ou 4.

6. Acide lipoïque, pouvant être obtenu selon l'une des revendications 2 à 4.

7. Utilisation d'acide lipoïque selon la revendication 1, 5 ou 6 pour la préparation de médicaments.

8. Utilisation d'acide lipoïque selon la revendication 1, 5 ou 6 comme additif dans des aliments ou comme composant dans des préparations de complément alimentaire.
